# EUROPÄISCHE PATENTSCHRIFT

(11) **EP 1 543 935 B1**
(45) Veröffentlichungstag und Bekanntmachung des Hinweises auf die Patenterteilung: **19.03.2014**
(21) Anmeldenummer: 04029290.6
(22) Anmeldetag: 10.12.2004
(51) Int. Cl.: B29C 45/14, A61B 5/00

(54) **Kunststoff-Spritzgussteil mit eingebettetem Bauteil**
Injection moulded plastic article with embedded part
Article moulé par injection avec pièce intégrée

(30) Priorität: 17.12.2003 DE 10359303
(43) Veröffentlichungstag der Anmeldung: 22.06.2005
(60) Teilanmeldung: 11189497.8
(73) Patentinhaber: F. Hoffmann-La Roche AG, 4070 Basel (CH); Roche Diagnostics GmbH, 68305 Mannheim (DE)
(72) Erfinder: Augstein, Manfred, 68305 Mannheim (DE)
(74) Vertreter: Isenbruck, Günter

(56) Entgegenhaltungen:
- EP-A- 0 283 285
- EP-A- 1 203 542
- DE-U1- 7 624 914
- US-A- 5 757 666

## Beschreibung

Die Erfindung bezieht sich auf ein Verfahren zur Herstellung eines Kunststoffspritzgussteiles mit einem in dieses eingespritzten Bauteiles aus Keramik oder Glas, wobei es sich bei dem Kunststoffspritzgussteil um einen Schalenkörper eines Schnelldiagnosegerätes handeln kann.

### Stand der Technik

Bei Schnelldiagnosegeräten, wie sie zur Blutzuckerbestimmung oder zur Bestimmung anderer Blutwerte eingesetzt werden, ist oftmals eine Temperierung des Reagenzbereiches innerhalb des Gerätegehäuses des Schnelldiagnosegerätes erforderlich. In den Auswertebereich eines Schnelldiagnosegerätes werden beispielsweise mit menschlichem oder tierischem Blut benetzte Teststreifen eingeführt. Es ist auch möglich, zunächst den Teststreifen in das Schnelldiagnosegerät einzuführen und erst dann die Probe auf den Teststreifen aufzubringen. Die Teststreifen enthalten Substanzen, welche mit dem Bereich des Teststreifens, der mit Blut menschlichen oder tierischen Ursprungs benetzt ist, reagieren. Zur Durchführung eines ein aussagekräftiges Messergebnis liefernden Messvorganges ist eine bestimmte Temperatur während des Auswertevorganges erforderlich.

Zur Erzeugung eines entsprechenden Temperaturniveaus werden daher metallische oder keramische Heizelemente in das Innere des Gehäusekörpers des Schnelldiagnosegerätes integriert. Diese Heizelemente werden im Normalfall in der kritischen Systemumgebung in Kunststoffbauteile eingebettet. Die Einbettung der Heizelemente aus metallischen oder keramischen Werkstoffen erfolgt in der Regel durch eine mechanische Befestigung mittels Feder oder Klemmelementen oder auch durch Einkleben des Heizelementes aus metallischem oder keramischem Werkstoff in eine dafür im Kunststoffmaterial vorgesehene Vertiefung.

Die Nachteile einer mechanischen Befestigung des Heizelementes aus keramischem oder metallischem Material innerhalb eines Kunststoffbauteiles sind darin zu erblicken, dass die Federkräfte auf das Heizelement ausübenden Federelemente bzw. Klemmelemente Spannungen in einem beispielsweise aus Keramik gefertigten Heizelement induzieren können, welches zu dessen Bruch führen kann, so dass das in das Schnelldiagnosegerät beispielsweise integrierte Heizelement unbrauchbar wird. Ferner kann es aufgrund der sich bei mechanischer Montage des Heizelementes im Gehäuseinneren ergebenden Fugen aufgrund von Fertigungstoleranzen - dazu kommen, dass bei einem übermäßig mit Blut benetzten Teststreifen das Blut durch die Fugen ins Geräteinnere gelangt und dort Beschädigungen an der Auswerteelektronik hervorruft. Gleiches gilt für ein Reinigungsmittel, mit welchem das Geräteinnere nach mehreren Teststreifenauswertungen nochmals gereinigt wird, um getrocknetes Blutplasma, welches sich beispielsweise an aus metallischem oder keramischem Werkstoff beschaffenem Heizelement angelagert hat, zu entfernen. Die eingesetzten Reinigungsmittel sind oft recht aggressiv, um das am Heizelement aus metallischem oder keramischem Material angelagerte meist flächig anhaftende Blutplasma anzulösen und entfernen zu können. Tritt das Reinigungsmittel, welches oft ein extremes Anlöseverhalten aufweist, durch die sich bei einer mechanischen Montage des metallischen oder keramischen Heizelementes ergebenden Fugen in das Geräteinnere ein, so kann auch durch das Reinigungsmittel die Elektronik Schaden nehmen.

Die mechanische Befestigungsvariante eines Heizelementes aus keramischem oder metallischem Werkstoff innerhalb eines Schnelldiagnosegerätes weist zudem den Nachteil auf, dass hohe Montagekosten entstehen, wobei das Risiko einer möglichen Fehlmontage nicht unerheblich ist. Bei einer Fehlmontage eines nachträglich in ein Kunststoffbauteil einzubauenden Heizelementes kann es zu Temperierungsfehlern kommen, welche die erhaltenen Messresultate bei einer Auswertung eines in das Schnelldiagnosegeräte eingeführten Teststreifens oft unbrauchbar machen können.

Anstelle der mechanischen Befestigungsvariante kann das aus metallischem oder keramischem Werkstoff gefertigte Heizelement auch im Inneren des Schnelldiagnosegerätes in eine entsprechende Vertiefung einer Halbschale eingeklebt werden. Beim Einkleben eines aus metallischem oder keramischem Werkstoff gefertigten Heizelementes in eine Ausnehmung im Inneren des Schnelldiagnosegerätes können zwar die bei der oben genannten Befestigungsvariante auftretenden Fugen weitestgehend vermieden werden, die dem Klebstoff beigemischten Lösungsmittel, können jedoch den in das Geräteinnere eingeführten Teststreifen beeinflussen. Ferner ist nicht auszuschließen, dass die Reinigungsmittel, mit denen das Heizelement zur Entfernung von getrocknetem Blutplasma von Zeit zu Zeit gereinigt wird, den Klebstoff, mit welchem das Heizelement in eine Vertiefung innerhalb des Gehäuseinneren eingeklebt ist, wieder anlösen. Ferner ist jeder Klebstoff über die Betriebszeit insbesondere bei starken Temperaturschwankungen einer Alterung unterworfen, welches diese Befestigungsvariante mit einem Risiko hinsichtlich der Zuverlässigkeit über die Betriebszeit eines Schnelldiagnosegerätes belastet.

Ferner ist auch gemäß dieser Befestigungsvariante der hohe Montageaufwand von Nachteil, wenn diese Variante in einer Großserienproduktion - wie beispielsweise einer Großserienproduktion von Schnelldiagnosegeräten - eingesetzt wird. Auch hier ist der Herstellungsprozess nicht frei von Montagefehlern, die gemäß des oben Gesagten die Aussagefähigkeit erhaltener Auswertungsergebnis erheblich in Frage stellen kann.

Eine weitere Befestigungsvariante zur Befestigung eines Bauteiles, wie beispielsweise eines aus metallischen oder keramischen Werkstoff gefertigten Heizelementes innerhalb eines Kunststoffspritzgießbauteiles, ist durch dessen direktes Einspritzen als Insert innerhalb des Spritzgießvorganges des Kunststoff-Spritzgußteiles gegeben. Das Problem, was sich gemäß dieses Herstellungsverfahrens stellt, liegt darin, dass der innerhalb des Spritzgießwerkzeuges auftretende Druck für bruchempfindliche Materialien, wie z.B. Keramik, problematisch ist, da bruchgefährdete Materialien wie Keramik oder Glas nicht beliebig gepresst werden können.

Ein Verfahren zur Herstellung eines scheibenförmigen Kunststoffkörpers mit einem eingeschlossenen Einlegeteil aus einem anderen Material ist aus der EP 1 203 542 A1 bekannt. Der Kunststoffkörper umfasst einen Füllkörper, der das Einlegeteil aufnimmt. Der Füllkörper ist zweiteilig ausgeführt und wird von zwei deckelartigen Folien nach außen begrenzt. Die Teile des Füllkörpers können im Kunststoff-Spritzgussverfahren hergestellt werden. Während des Spritzgussvorgangs erfolgt eine dichte, fugenfreie Verbindung zwischen Füllkörper und Folie. Auch das Einlegeteil kann dabei mit dem Füllkörper verbunden werden. Die beiden Teile des Füllkörpers werden formschlüssig miteinander verbunden und/oder verzahnt. Die Verzahnung kann ebenfalls durch Spritzgusstechnik mittels Kunststoffmasse erfolgen.

Angesichts der oben skizzierten, dem Stande der Technik anhaftenden Nachteile liegt der Erfindung die Aufgabe zugrunde, unterschiedliche, auch bruchgefährdete Materialien wie beispielsweise Glas oder Keramik im Spritzgussfertigungsprozess zu bearbeiten.

Erfindungsgemäß wird diese Aufgabe durch die Merkmale der Patentansprüche 1 und 13 gelöst.

Die mit dem erfindungsgemäß vorgeschlagenen Verfahren einhergehenden Vorteile sind vor allem darin zu erblicken, dass nunmehr innerhalb eines Arbeitsganges im Spritzgießprozess im Mehrkomponenten-Spritzgießverfahren sowohl das oder die Kunststoffmaterialien als auch bruchgefährdete Materialien wie Keramik oder Glas gleichzeitig verarbeitet werden können, da am Spritzgießwerkzeug, in welchem das Kunststoff-Spritzgussbauteil mit einem Insert aus bruchgefährdetem Material wie Keramik oder Glas umspritzt wird, ein federgesteuerter Schließmechanismus eingesetzt wird.

Somit besteht die Möglichkeit, am Spritzgießwerkzeug die maximale Presskraft auf die maximale Kraft einzustellen, mit welcher das bruchgefährdete Bauteil beaufschlagt werden kann. Dies eröffnet wiederum die Möglichkeit, an einem Spritzgießwerkzeug auch unterschiedliche bruchgefährdete Materialien als Inserts in Kunststoffbauteile einzubetten, die unterschiedliche Kräfte aufzunehmen vermögen. Die Maximalschließkraft kann somit jeweils auf den als Insert verwendeten Werkstoff individuell abgestimmt werden.

Optional kann das bruchgefährdete Bauteil mit einer Dämpfungsschicht beschichtet werden. Als Dämpfungsschicht lässt sich z.B. ein vollflächig auftragbarer Lack einsetzen, welcher entweder auf die bruchgefährdeten Bauteile oder auf die Stahlteile des Spritzgusswerkzeuges aufgetragen ist, die mit dem Insert aus bruchgefährdetem Material wie Keramik oder Glas in Kontakt kommen, was ansonsten zum Bruch des bruchgefährdeten Materials führen könnte. Es besteht die Möglichkeit, die vorgeschlagene - als Lackschicht ausgestaltbare - Dämpfungsschicht so aufzubringen, dass das in das Kunststoffmaterial einzubettende Insert vollständig von dieser Dämpfungsschicht umgeben ist. Andererseits ist es möglich, die Dämpfungsschicht als Rahmen um das bruchgefährdete, als Insert in das Kunststoffmaterial einzubettende Bauteil aus bruchgefährdetem Material aufzubringen, so dass nur der Kontakt der Stahlteile des Spritzwerkzeuges an bestimmten Stellen mit dem Insert aus bruchgefährdetem Material gedämpft wird.

Werden beispielsweise "Analysechips", insbesondere sogenannte ("Biochips") mit als Inserts in Kunststoffmaterial im Wege des erfindungsgemäß vorgeschlagenen Spritzgießverfahrens hergestellt, so lassen sich diese mit einem Kunststoffrahmen umspritzen, wobei bei der Herstellung von Biochips lediglich teilweise eine Dämpfungsschicht aufzubringen ist und andere Bereiche des Glases, welches ebenfalls ein bruchgefährdetes Material darstellt, unbehandelt verbleiben könnten. Die unbehandelten Partien des Glases könnten nachträglich mit entsprechenden Reagenzien, die zum Einsatz des Inserts mit einem Glasträger als Grundmaterial, erforderlich sind, belegt werden.

Das erfindungsgemäß vorgeschlagene Herstellverfahren zur Herstellung eines in ein Kunststoff-Bauteil integriertes Insert aus bruchgefährdetem Material wie beispielsweise Keramik oder Glas ist durch eine hohe Prozesssicherheit gekennzeichnet. Da es sich um einen werkzeuggebundenen, nämlich an das Spritzgießwerkzeug gekoppelten Herstellungsprozess handelt, entfallen Montagekosten vollständig sowie das damit verbundene Risiko der Fehlmontage. Mit dem erfindungsgemäß vorgeschlagenen Herstellungsverfahrens zur Einbettung eines Inserts aus bruchgefährdetem Material in ein spritzzugießendes Kunststoff-Spritzgießbauteil ist eine fugenlose Einbettung eines Heizelementes aus metallischem oder keramischem Material in eine Kunststoffschale des Kunststoffgehäuses eines Schnelldiagnosegerätes problemlos möglich. Aufgrund der fugenlosen Einbettung eines Heizelementes aus keramischem oder metallischem Material in einem gleichen Arbeitsgang herzustellendes Kunststoff-Spritzgießbauteil wird eine flüssigkeitsdichte, formschlüssige Einbettung des Inserts in das Kunststoffmaterial erreicht.

### Zeichnung

Anhand der Zeichnung wird die Erfindung nachstehend eingehender beschrieben.

Es zeigt:
- Figur 1: eine perspektivische Ansicht eines Schnelldiagnosemessgerätes,
- Figur 2: eine offen liegende Gehäuseöffnung eines Schnelldiagnosegerätes,
- Figur 3: einen Ausschnitt aus dem Gehäusekörper eines Schnelldiagnosemessgerätes gemäß der Darstellung in Figur 1 mit einem integrierten Heizelement aus einem bruchgefährdeten Material wie beispielsweise Keramik,
- Figur 4: ein schematisch dargestelltes Spritzgießwerkzeug mit variabler Schließkrafteinstellung,
- Figur 5: einen Glaskörper, der von einem Rahmen aus Kunststoffmaterial umschlossen ist,
- Figur 6: einen Keramikkörper, der auf seiner Oberseite mit einer als Dämpfungsschicht dienenden Lackbeschichtung versehen ist und
- Figur 7: ein Insert aus keramischem Material, welches fugenlos in ein Kunststoffmaterial eingebettet ist.

### Ausführungsvarianten

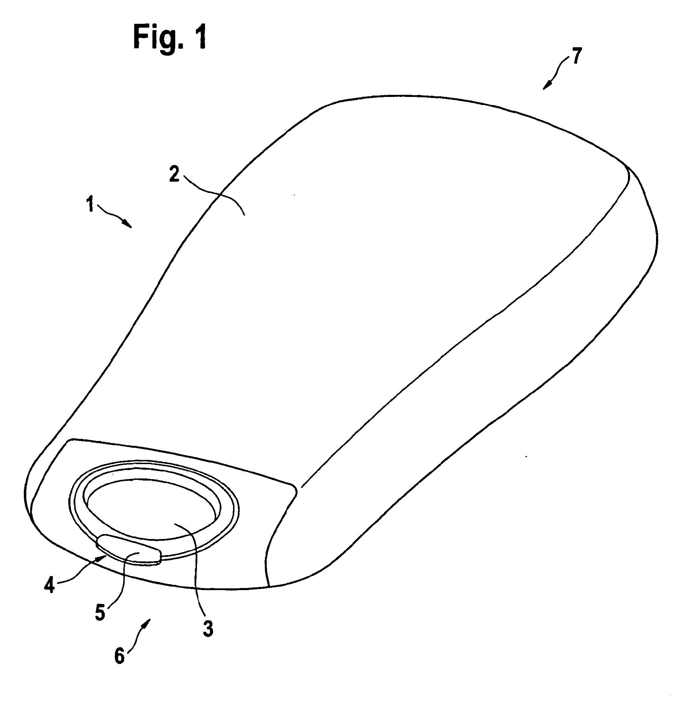

Der Darstellung gemäß Figur 1 ist in perspektivischer Ansicht ein Schnelldiagnosegerät zu entnehmen, dessen Gehäusekörper aus einem Kunststoffmaterial spritzgegossen ist.

Ein in Figur 1 dargestelltes Schnelldiagnosegerät 1 umfasst einen Gehäusekörper 2, der aus einem Kunststoffmaterial gefertigt ist. An einer Vorderseite 6 des Gehäusekörpers 2 ist ein Deckelelement 3 vorgesehen, dessen untere Berandung oberhalb einer Einschuböffnung 4 liegt. Die Einschuböffnung 4 umfasst als Auflagefläche für einen in das Innere des Gehäusekörpers 2 einzuschiebenden Teststreifen eine Einschubzunge 5. Die Rückseite des Gehäusekörpers 2 des Schnelldiagnosegerätes 1 ist mit Bezugszeichen 7 gekennzeichnet. Das Schnelldiagnosegerät 1 dient der Auswertung von in den Gehäusekörper 2 einzuschiebenden Teststreifen, die mit Blut menschlichen oder tierischen Ursprungs benetzt sind. Die Teststreifen weisen chemische Substanzen auf, die mit dem benetzten Blutvorrat reagieren, um beispielsweise eine Blutzuckermessung vorzunehmen. Im Gehäusekörper 2 sind zur Durchführung der Messung am Teststreifen sowohl Heizelemente, elektrische Kontaktierungselemente für den in die Einschuböffnung 4 einzuschiebenden Teststreifen als auch eine Auswerteelektronik integriert sowie ein optisches Display angeordnet.

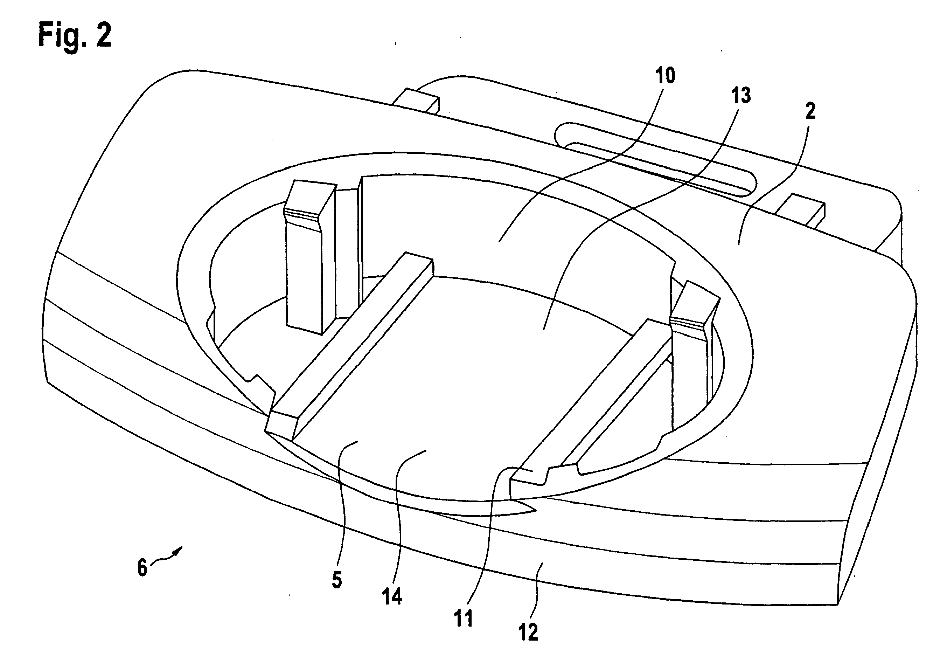

Der Darstellung gemäß Figur 2 ist die Vorderseite 6 des Gehäusekörpers 2 des Schnelldiagnosegerätes 1 zu entnehmen. In der Vorderseite 6 des Gehäusekörpers 2 ist eine Gehäuseöffnung 10 ausgebildet, die ein in Figur 2 dargestelltes ovales abgerundetes Aussehen aufweisen kann. Die Unterseite der Gehäuseöffnung 10 wird durch die Einschubzunge 5 begrenzt. Beidseits der Einschubzunge 5 sind erhaben hervorstehende Einschubschienen 11 ausgebildet, zwischen denen ein in die Einschuböffnung 4 einzuführender Teststreifen (in Figur 2 nicht dargestellt) in das Gehäuseinnere 13 des Gehäusekörpers 2 einschiebbar ist. Der in die Einschuböffnung 4 einzuführende Teststreifen wird einerseits durch die beiden Einschubschienen 11 und andererseits durch die Oberseite 14 der Einschubzunge 5 geführt.

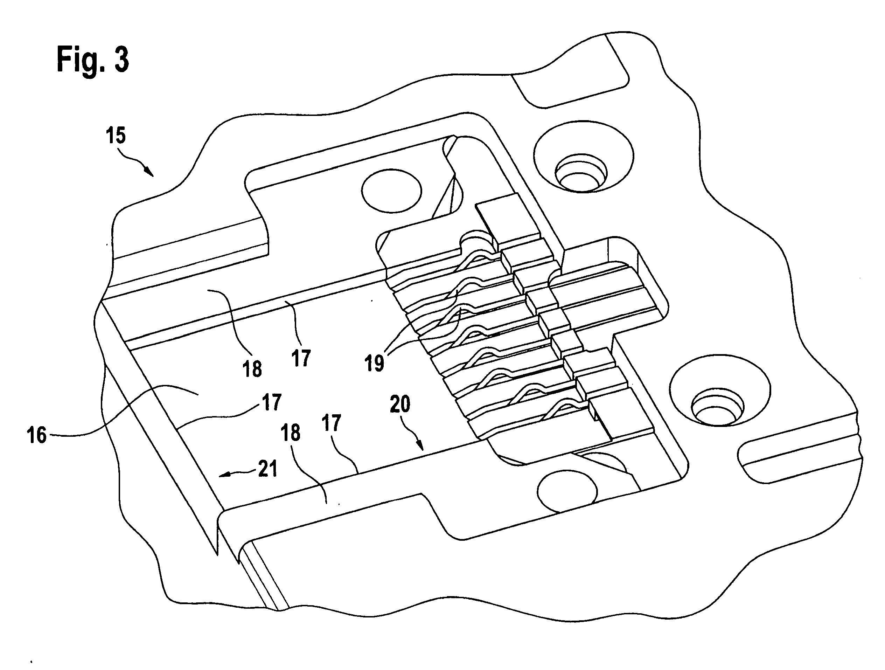

Figur 3 ist ein systemkritischer Bereich im Inneren des Gehäusekörpers eines Schnelldiagnosegerätes beispielsweise zu entnehmen.

Innerhalb eines durch das Bezugszeichen 15 definierten kritischen Systembereiches innerhalb des Gehäusekörpers 2 ist ein Insert 16 in ein beispielsweise als Gehäuseunterschale spritzgegossenes Kunststoff-Bauteil integriert. Im Falle eines Schnelldiagnosegerätes 1 handelt es sich bei dem in das Kunststoffmaterial eingebetteten Insert 16 um ein Heizelement, welches aus einem bruchgefährdeten Material wie Keramik oder auch aus Metall gefertigt sein kann. Das als Heizelement ausgebildete Insert 16 erzeugt im Inneren des Gehäusekörpers 2 bei eingeschobenen Teststreifen eine Temperatur, bei welcher der Reagenzbereich am eingeführten Teststreifen auf eine Temperatur gebracht wird, welcher eine aussagekräftige Messung innerhalb eines Schnelldiagnosegerätes ermöglicht.

In der Darstellung gemäß Figur 3 ist das Insert 16 mit einer fugenfreien Einbettung 17 in die Unterschale des Gehäusekörpers 2 integriert. Der in Figur 3 nicht dargestellte Teststreifen überdeckt das als Heizelement ausgebildete Insert 16 an dessen Oberseite und kann über elektrische Kontaktierungen 19 kontaktiert werden. Eine Längsseite 20 des Inserts 16 verläuft parallel zur Messkante eines Messstreifens, während die kürzer ausgebildete Querseite 21 des Inserts 16 senkrecht zu dessen Einschubrichtung in das Innere des Gehäusekörpers 2 verläuft.

Der Teststreifen, der im in den Gehäusekörper 2 eingeschobenen Zustand das Insert 16 überdeckt, wird durch das als Heizelement ausgebildete Insert 16 aus keramischem Material beheizt und auf eine zu einem aussagekräftigen Messergebnis erforderliche Temperatur gebracht. Diese ist abhängig von den Reagenzien, welche im Teststreifen vorgesehen sind.

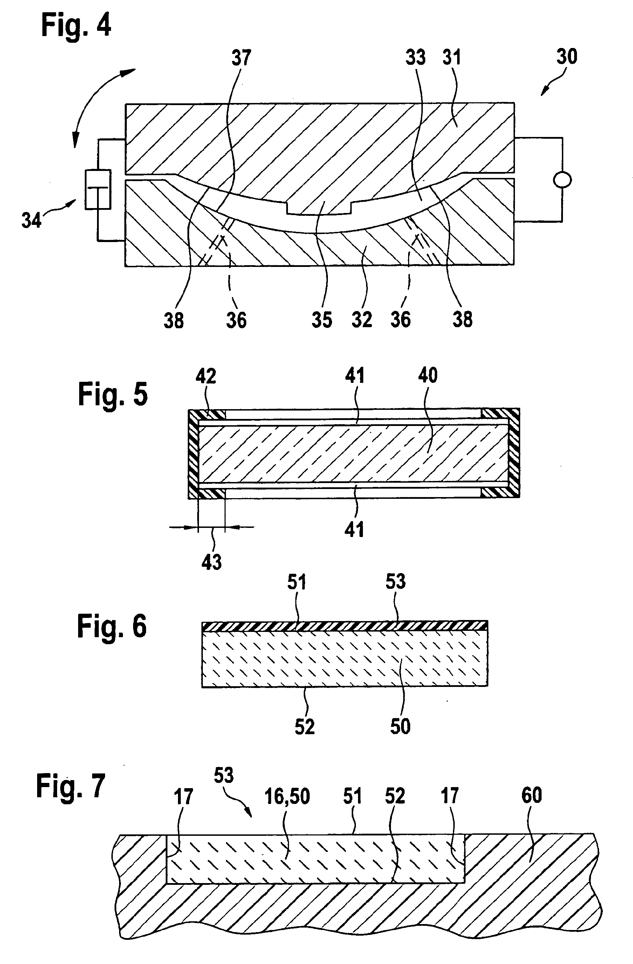

Figur 4 ist in schematischer Wiedergabe ein Spritzgießwerkzeug zu entnehmen, in dessen Kavität ein Kunststoffmaterial und ein bruchgefährdetes Material gleichzeitig spritzgegossen werden können.

Der Darstellung gemäß Figur 4 ist ein Spritzgießwerkzeug 30 entnehmbar, welches eine erste Werkzeughälfte 31 und eine zweite Werkzeughälfte 32 aufweist. Die erste Werkzeughälfte 31 ist relativ zur zweiten Werkzeughälfte 32 in Richtung des Doppelpfeiles verschiebbar, d.h. öffenbar und schließbar. Die erste Werkzeughälfte 31 und die zweite Werkzeughälfte 32 begrenzen eine Kavität 33. Im geschlossenen Zustand der ersten Werkzeughälfte 31 und der zweiten Werkzeughälfte 32 sind diese über einen Schließmechanismus verriegelt. Die Andruckkraft, die am verstellbar ausgebildeten Andruckmechanismus eingestellt werden kann, ist abhängig von der Kraft, welcher das bruchgefährdete Material, aus welchem das Insert 16 gefertigt ist, gerade noch standhält.

Die erste Werkzeughälfte 31 und die zweite Werkzeughälfte 32 sind über ein Gelenk miteinander verbunden. In der ersten Werkzeughälfte 31 oder in der zweiten Werkzeughälfte 32 können Angusskanäle 36 vorgesehen werden, über welche das Kunststoffmaterial in die durch die Werkzeughälften 31 und 32 gebildete Kavität 33 einströmt. Das Insert 16 wird durch einen Andruckstempel 35 aufgenommen und positioniert. Beim Zufahren der Werkzeughälften 31, 32 wird das Insert 16 plan gegen die erste Werkzeughälfte 31 gepresst und damit gehalten. Die Ausnehmung zur Einbettung des Inserts 16 wird durch das Einfügen des Inserts 16 in die Kavität 33 und deren anschließendes Umspritzen erzeugt.

Der Darstellung gemäß Figur 5 ist ein Insert 16 zu entnehmen, welches aus Glas 40 beschaffen ist. Das als Glaskörper 40 beschaffene Insert 16 ist an seiner Umfangsfläche von einem Kunststoffrahmen 42 umgeben. Der Kunststoffrahmen 42 weist an seinen Längsseiten Überstände 43 auf, so dass ein nicht unerheblicher Teil der Ober- und der Unterseite des Glaskörpers 40 als Freifläche 41 verbleibt. Die Umrahmung des Glaskörpers 40 mit einem Kunststoffrahmen 42 bietet sich insbesondere bei der Herstellung von Bio-Chips an, deren Oberseiten nachträglich mit entsprechenden Reagenzien, die zum Einsatz von Bio-Chips erforderlich sind, belegt oder beschichtet werden können. Durch den den Glaskörper 40 umschließenden Kunststoffrahmen 42 kann erreicht werden, dass ein Kontakt der Stahlteile der Formenhälften 31 bzw. 32 mit dem überaus bruchgefährdeten Glaskörper 40 vermieden werden kann, in dem die Stahlteile der ersten und der zweiten Formenhälfte 31 bzw. 32 lediglich die Außenseite des den Glaskörper 40 umschließenden Kunststoffrahmen 42 kontaktieren.

Figur 6 zeigt ein als Keramikkörper 50 beschaffenes Insert 16, auf dessen Oberseite 51 eine vollflächige Dämpfungsschicht 53 in Form einer Lackschicht aufgebracht ist. In der Darstellung gemäß Figur 6 ist die Unterseite 52 des Keramikkörpers 50 unbehandelt. Neben einer in Figur 6 dargestellten vollflächigen Beschichtung mit einer Lackschicht 53 als Dämpfungsschicht können auch Teilbereiche des Keramikkörpers 50 sowohl an dessen Oberseite 51 als auch an dessen Unterseite 52 mit einer als Lackschicht beschaffenen Dämpfungsschicht beschichtet werden.

Aus der Darstellung gemäß Figur 7 geht hervor, dass ein Insert, welches beispielsweise aus einem keramischen Material oder aus einem metallischen Material gefertigt werden kann, in ein Kunststoffmaterial 60 fugenfrei eingebettet ist. Das in Figur 7 dargestellte Insert 16, 50 kann als Heizelement in einen Gehäusekörper 2 eines Schnelldiagnosegerätes (vergleiche Ausschnitt gemäß Figur 3) fugenfrei eingebettet sein und innerhalb des systemkritischen Bereiches 15 vorgesehen sein. Unter systemkritischer Bereich 15 wird der Bereich innerhalb eines Schnelldiagnosegerätes 1 verstanden, an welchem aus einem in das Innere des Schnelldiagnosegerätes 1 eingeführten Teststreifens überschüssiges Blut bzw. aggressive Reinigungsmittel, mit welchen das Gehäuseinnere des Schnelldiagnosegerätes 1 von Zeit zu Zeit gereinigt wird, vorhanden sein können.

Durch die erfindungsgemäß vorgeschlagene Ausgestaltung des Inserts 16 als integraler Bestandteil eines aus Kunststoffmaterial 60 gefertigten Gehäusekörpers 2 werden die Fugen zwischen den Inserts 16, 50 und dem Kunststoffmaterial 60, die beim Einkleben bzw. beim mechanischen Arretieren des Inserts 16, 50 auftreten, durch die erfindungsgemäß vorgeschlagene Lösung vermieden. Durch die erfindungsgemäß vorgeschlagene Lösung wird insbesondere erreicht, dass die Oberseite des Inserts 16, 50 mit der Oberseite des Kunststoffmaterials 60 eine einheitliche Ebene bildet, wodurch das Einführen eines Teststreifens an der Einschuböffnung 4 in das Gehäuseinnere des Gehäusekörpers 2 eines Schnelldiagnosegerätes 1 erleichtert wird.

Bei dem erfindungsgemäß vorgeschlagenen Herstellungsverfahren eines Kunststoffbauteiles mit im gleichen Arbeitsgang eingebettetem Insert aus einem bruchgefährdeten Material kann ein einfacher werkzeuggebundener Herstellprozess bereitgestellt werden, der sich insbesondere durch eine hohe Prozesssicherheit auszeichnet. Fehlmontagen, wie sie bei den aus dem Stand der Technik bekannten Montageverfahren herrühren, können ausgeschlossen werden. Innerhalb des systemkritischen Bereiches 15, der durch aggressive Reinigungsmedien bzw. durch Blutplasmaablagerungen kontaminiert sein kann, ist eine fugenlose Einbettung eines aus keramischen, d.h. einem bruchgefährdeten Material gefertigten Inserts in den Einschubbereich eines Teststreifens möglich, der sich durch eine fugenlose Einbettung in das Kunststoffmaterial 60 auszeichnet. Eine unter dem Insert 16, 50 aus keramischen Material liegende Elektronik wird aufgrund des Fehlens ein Durchsickern von Flüssigkeit ermöglichender Fugen nach dem nunmehr vorgeschlagenen Verfahren wirksam gegen diese Medien geschützt. Eine hohe Kosten verursachende nachträglich Montage eines Heizelementes in ein Schnelldiagnosegerät beispielsweise, wie es bisher Stand der Technik war, kann nunmehr entfallen. Die fugenlose Einbettung 17 des Inserts 16, 50 aus einem bruchgefährdeten Material wie beispielsweise Metall oder Keramik ermöglicht weiterhin in vorteilhafter Weise eine flüssigkeitsdichte formschlüssige Verbindung zwischen dem Kunststoffmaterial 60 des Gehäusekörpers 2 und dem Insert 16, 50 aus dem bruchgefährdeten Material, welches entlang seiner Längsseite 20 und entlang seiner Querseite 21 (vergleiche Darstellung gemäß Figur 3) fugenlos in das Kunststoffmaterial 60 zwischen zwei Führungsschienen zur Führung des eines in das Gehäuseinnere einzuschiebende Teststreifens, eingebettet ist.

### Bezugszeichenliste

- 1: Schnelldiagnosegerät
- 2: Gehäusekörper
- 3: Deckel
- 4: Einschuböffnung
- 5: Einschubzunge
- 6: Vorderseite
- 7: Rückseite

- 10: Gehäuseöffnung
- 11: Einschubschiene Teststreifen
- 12: Gehäuseunterschale
- 13: Gehäuseinneres
- 14: Oberseite Einschubzunge
- 15: kritischer Systembereich
- 16: Insert
- 17: fugenfreie Einbettung
- 18: Teststreifenführung
- 19: elektrische Kontaktierung
- 20: Längsseite Insert
- 21: Querseite Insert

- 30: Spritzgießwerkzeug
- 31: erste Werkzeughälfte
- 32: zweite Werkzeughälfte
- 33: Kavität
- 34: Schließmechanismus
- 35: Andruckstempel

- 36: Angusskanal

- 40: Insert aus Glas
- 41: Freifläche
- 42: Einfassung aus Kunststoffmaterial
- 43: Überstand

- 50: Insert aus Keramik
- 51: Oberseite
- 52: Unterseite
- 53: Dämpfungsschicht (Lack)

- 60: Kunststoffmaterial

## Patentansprüche

1. Verfahren zur Herstellung eines Kunststoff-Spritzgußteiles (2) als Gehäusekörper für ein Schnelldiagnosegerät mit einem Insert (16, 40, 50) aus einem vom Kunststoffmaterial (60) verschiedenen Material mit nachfolgenden Verfahrensschritten:
a) Einbringen und Positionieren des Inserts (16, 40, 50) in eine Kavität (33) eines Spritzgießwerkzeuges (30, 31, 32), wobei das aus einem bruchgefährdetem Material bestehende Insert mit einer Dämpfungsschicht (53) vorbehandelt wird,
b) Einstellen der Schließkraft des Spritzgießwerkzeuges (30, 31, 32) auf eine vom Material des Inserts (16, 40, 50) vorgegebene maximale Kraft an einem Schließmechanismus (34),
c) teilweises fugenfreies Umspritzen des Inserts (16, 40, 50) mit dem Kunststoffinaterial (60) des Kunststoff-Spritzgußteiles (2) innerhalb des Spritzgießwerkzeuges (30, 31, 32).

2. Verfahren gemäß Anspruch 1, **dadurch gekennzeichnet, dass** das Insert (16, 40, 50) an mindestens einer einer der Werkzeughälften (31, 32) des Spritzgusswerkzeuges (30) zuweisenden Seite (51, 52) mit der Dämpfungsschicht (53) versehen wird.

3. Verfahren gemäß Anspruch 1, **dadurch gekennzeichnet, dass** das Insert (16, 40, 50) mit einer vollflächigen Dämpfungsschicht (53) versehen wird.

4. Verfahren gemäß Anspruch 1, **dadurch gekennzeichnet, dass** das Insert (16, 40, 50) mit einer in Teilbereichen aufgebrachten Dämpfungsschicht (53) versehen wird.

5. Verfahren gemäß Anspruch 1, **dadurch gekennzeichnet, dass** die Dämpfungsschicht (53) als Lackschicht aufgetragen wird.

6. Verfahren gemäß Anspruch 1, **dadurch gekennzeichnet, dass** die Dämpfungsschicht (53) in Folienform oder lackförmig aufgetragen wird.

7. Verfahren gemäß Anspruch 1, **dadurch gekennzeichnet, dass** das Insert (16, 40, 50) innerhalb der Kavität (33) des Spritzgießwerkzeuges (30, 31, 32) mit einer Einfassung (42) aus Kunststoffmaterial (60) fugenfrei umspritzt wird.

8. Verfahren gemäß Anspruch 8, **dadurch gekennzeichnet, dass** die Einfassung (42) des Inserts (16, 40, 50) die Begrenzung einer Ausnehmung zur Einbettung des Inserts (16, 40, 50) in einen Gehäusekörper (2) bildet.

9. Verfahren gemäß Anspruch 2, **dadurch gekennzeichnet, dass** die Dämpfungsschicht (53) das Insert (16, 40, 50) vollständig umschließt.

10. Verfahren gemäß Anspruch 1, **dadurch gekennzeichnet, dass** als Insert (16, 40, 50) Bauteile aus Keramikmaterial eingesetzt werden.

11. Verfahren gemäß Anspruch 1, **dadurch gekennzeichnet, dass** als Insert (40) Bauteile aus Glas eingesetzt werden.

12. Schnelldiagnosegerät mit einem Gehäusekörper (2), wobei zumindest ein Teil des Gehäusekörpers gemäß einem oder mehrerer der Patentansprüche 1 bis 11 hergestellt wird, zur Auswertung eines Teststreifens, **dadurch gekennzeichnet, dass** in einem systemkritischen Bereich (15) innerhalb des Gehäusekörpers (2) aus Kunststoffmaterial (60) ein als Heizelement wirkendes Insert (16) aus bruchgefährdetem Material in fugenfreier Einbettung (17) eingelassen ist, wobei die Oberfläche des als Heizelement wirkenden Inserts teilweise frei bleibt.

13. Schnelldiagnosegerät gemäß Anspruch 12, **dadurch gekennzeichnet, dass** das als Heizelement fungierende Insert (16) aus bruchgefährdetem Material integraler Bestandteil zumindest eines Teils des Gehäusekörpers (2) ist.

14. Schnelldiagnosegerät gemäß Anspruch 12, **dadurch gekennzeichnet, dass** das als Heizelement fungierende Insert (16) aus bruchgefährdetem Material im Teil des Gehäusekörpers (2) im Formschluss aufgenommen ist.

15. Schnelldiagnosegerät gemäß Anspruch 12, **dadurch gekennzeichnet, dass** das Insert (16) aus bruchgefährdetem Material aus keramischen oder metallischen Material gefertigt ist.

## Claims

1. Method for production of a plastic injection-moulded part (2) as housing for a rapid diagnosis appliance, having an insert (16, 40, 50) made of a material different from the plastic material (60), said method comprising the following method steps:
(a) introduction and positioning of the insert (16, 40, 50) in a cavity (33) of an injection mould (30, 31, 32), wherein the insert, consisting of a breakable material, is pretreated with a damping layer (53),
(b) setting the clamping force of the injection mould (30, 31, 32), on a clamping mechanism (34), to a maximum force predetermined by the material of the insert (16, 40, 50),
(c) partial seamless encapsulation of the insert (16, 40, 50) with the plastic material (60) of the plastic injection-moulded part (2) inside the injection mould (30, 31, 32).

2. Method according to Claim 1, **characterized in that** the insert (16, 40, 50) is provided with the damping layer (53) on at least one side (51, 52) facing towards one of the mould halves (31, 32) of the injection mould (30).

3. Method according to Claim 1, **characterized in that** the insert (16, 40, 50) is provided with a damping layer (53) over its entire surface.

4. Method according to Claim 1, **characterized in that** the insert (16, 40, 50) is provided with a damping layer (53) applied in only some areas.

5. Method according to Claim 1, **characterized in that** the damping layer (53) is applied as a lacquer layer.

6. Method according to Claim 1, **characterized in that** the damping layer (53) is applied in the form of a film or in the form of a lacquer.

7. Method according to Claim 1, **characterized in that** the insert (16, 40, 50) inside the cavity (33) of the injection mould (30, 31, 32) is encapsulated seamlessly with a border (42) of plastic material (60).

8. Method according to Claim 7, **characterized in that** the border (42) of the insert (16, 40, 50) forms the limit of a recess for embedding the insert (16, 40, 50) in a housing body (2).

9. Method according to Claim 2, **characterized in that** the damping layer (53) completely surrounds the insert (16, 40, 50).

10. Method according to Claim 1, **characterized in that** components made of ceramic material are used as insert (16, 40, 50).

11. Method according to Claim 1, **characterized in that** components made of glass are used as insert (40).

12. Rapid diagnosis appliance for evaluation of a test strip, having a housing body (2), at least part of the housing body being produced in accordance with one or more of Patent Claims 1 to 11, **characterized in that** an insert (16) made of breakable material and acting as heating element is seamlessly embedded (17) in a system-critical area (15) inside the housing body (2) made of plastic material (60), wherein the surface of the insert acting as heating element remains partially free.

13. Rapid diagnosis appliance according to Claim 12, **characterized in that** the insert (16) made of breakable material and functioning as heating element is an integral component of at least part of the housing body (2).

14. Rapid diagnosis appliance according to Claim 12, **characterized in that** the insert (16) made of breakable material and functioning as heating element is received with a form fit in the part of the housing body (2).

15. Rapid diagnosis appliance according to Claim 12, **characterized in that** the insert (16) made of breakable material is made of ceramic or metal material.

## Revendications

1. Procédé de fabrication d'une pièce moulée par injection de plastique (2) en tant que corps de boîtier pour un appareil de diagnostic rapide, comprenant un insert (16, 40, 50) en matériau différent du matériau plastique (60), comprenant les étapes de procédé suivantes :
a) introduction et positionnement de l'insert (16, 40, 50) dans une cavité (33) d'un outil de moulage par injection (30, 31, 32), l'insert, constitué d'un matériau susceptible de rupture, étant prétraité avec une couche d'amortissement (53),
b) ajustement de la force de fermeture de l'outil de moulage par injection (30, 31, 32) à une force maximale prédéfinie par le matériau de l'insert (16, 40, 50) au niveau d'un mécanisme de fermeture (34),
c) surmoulage partiel sans joint de l'insert (16, 40, 50) avec le matériau plastique (60) de la pièce moulée par injection de plastique (2) à l'intérieur de l'outil de moulage par injection (30, 31, 32).

2. Procédé selon la revendication 1, **caractérisé en ce que** l'insert (16, 40, 50) est pourvu, au niveau d'au moins un côté (51, 52) tourné vers l'une des moitiés d'outil (31, 32) de l'outil de moulage par injection (30), de la couche d'amortissement (53).

3. Procédé selon la revendication 1, **caractérisé en ce que** l'insert (16, 40, 50) est pourvu d'une couche d'amortissement sur toute la surface (53).

4. Procédé selon la revendication 1, **caractérisé en ce que** l'insert (16, 40, 50) est pourvu d'une couche d'amortissement (53) appliquée dans des régions partielles.

5. Procédé selon la revendication 1, **caractérisé en ce que** la couche d'amortissement (53) est appliquée sous forme de couche de laque.

6. Procédé selon la revendication 1, **caractérisé en ce que** la couche d'amortissement (53) est appliquée sous forme de film ou sous forme de laque.

7. Procédé selon la revendication 1, **caractérisé en ce que** l'insert (16, 40, 50) est surmoulé sans joint à l'intérieur de la cavité (33) de l'outil de moulage par injection (30, 31, 32) avec une monture (42) en matériau plastique (60).

8. Procédé selon la revendication 7, **caractérisé en ce que** la monture (42) de l'insert (16, 40, 50) forme la limitation d'un évidement pour l'encastrement de l'insert (16, 40, 50) dans un corps de boîtier (2).

9. Procédé selon la revendication 2, **caractérisé en ce que** la couche d'amortissement (53) entoure complètement l'insert (16, 40, 50).

10. Procédé selon la revendication 1, **caractérisé en ce que** l'on utilise en tant qu'insert (16, 40, 50) des composants en matériau céramique.

11. Procédé selon la revendication 1, **caractérisé en ce que** l'on utilise en tant qu'insert (40) des composants en verre.

12. Appareil de diagnostic rapide comprenant un corps de boîtier (2), au moins une partie du corps de boîtier étant fabriquée selon l'une ou plusieurs des revendications 1 à 11, pour l'analyse d'une bandelette de test, **caractérisé en ce que** dans une région critique du système (15) à l'intérieur du corps de boîtier (2) en matériau plastique (60), est incorporé un insert (16) servant d'élément chauffant en matériau susceptible de rupture, dans un encastrement sans joint (17), la surface de l'insert servant d'élément chauffant restant partiellement exposée.

13. Appareil de diagnostic rapide selon la revendication 12, **caractérisé en ce que** l'insert (16) servant d'élément chauffant en matériau susceptible de rupture fait partie intégrante d'au moins une partie du corps de boîtier (2).

14. Appareil de diagnostic rapide selon la revendication 12, **caractérisé en ce que** l'insert (16) servant d'élément chauffant en matériau susceptible de rupture est reçu dans la partie du corps de boîtier (2) par engagement par correspondance de forme.

15. Appareil de diagnostic rapide selon la revendication 12, **caractérisé en ce que** l'insert (16) matériau susceptible de rupture est fabriqué en matériau céramique ou métallique.
